# EUROPEAN PATENT SPECIFICATION

(11) **EP 1 969 116 B1**
(45) Date of publication and mention of the grant of the patent: **25.11.2009**
(21) Application number: 06806908.7
(22) Date of filing: 29.09.2006
(51) Int. Cl.: C12M 1/24, B01L 3/14

(54) **LABORATORY CULTURE FLASK WITH SNAP-ON CAP**
LABORKULTURKOLBEN MIT SCHNAPPDECKEL
BALLON DE CULTURE DE LABORATOIRE AVEC CAPUCHON-PRESSION

(30) Priority: 22.12.2005 WO PCT/EP2005/057098
(43) Date of publication of application: 17.09.2008
(73) Proprietor: Pentacore International Holding, 1324 Almere (NL)
(72) Inventor: MENCHEL, Yariv, B-1420 Braine L'alleud (BE); RAM, Yoav, 30095 Ramat Ishai (IL); MENCHEL, Lyron, B-1348 Braine L'alleud (BE)
(74) Representative: Pecher, Nicolas
(86) International application number: PCT/EP2006/066942
(87) International publication number: WO 2007/071466

(56) References cited:
- US-A- 6 085 922
- US-A- 6 114 165

## Description

### Technical Field

The invention relates to the field of flasks. More particularly, the invention relates to a flask and cap for laboratory culture.

### Description of Related Art

Flasks for a wide range of applications, made of various materials, and having corresponding closure means have been designed and used. In the laboratory, culture flasks are used to culture microorganisms or tissues in a culture medium which adheres to an internal surface of the flask. It is a general requirement of such flasks that they provide a large accessible opening for inserting or removing material through a pipette or scraper. It is another general requirement that such flasks may be closed in either a vented or air-tight way.

A gas permeable sterile culture bottle is known from US 3,870,602. This bottle includes a neck opening arranged at an inclined angle with respect to the bottom wall so that a pipette or other instrument may be inserted into the bottle to reach any portion of the contents therein. The cylindrical neck is threaded and is adapted for receiving a threaded cap.

Another laboratory culture flask is known from US 4,770,854. A portion of the bottom wall of this flask defines an inclined ramp from the neck to a horizontal portion of the bottom wall. The neck is provided with a flat lower surface which intersects the ramp. The neck, circular at its open end, is provided with a thread for cooperating with a screw cap. The extra wide neck, and its flat lower surface connected to said ramp provide improved access to all four corners of the flask.

Still another tissue culture flask is known from US 5,924,583. This flask is also provided with a wide neck for improved access. This neck is obtained by raising the neck slightly above the upper wall of the flask, and by providing a filler wall extending from the neck to the flask front wall. A conventional threaded screw cap is provided for sealing the interior of the flask.

Document US 6,818,438 also discloses a tissue culture flask attempting to improve the inside accessibility. In this flask, the neck is circular at the open end, but is an upwardly concave elliptical arc and a downwardly concave circular arc at the end of the neck adjacent to the flask. The elliptical sections of the neck are substantially tangent to the ramp at the front wall of the flask. This geometry facilitates access by pipettes, scrapers and other instruments. The cylindrical open end of the flask is provided with a neck adapted for a screw-on cap.

In the course of laboratory experiments and tests, it is often required to open, put in venting position, or close a large number of flasks A venting position is especially desirable for tissue culture, where gases may be emitted or absorbed. A free exchange of gases is therefore necessary for limiting the pressure inside the flask, and providing oxygen or other gases to the inside of the flask. A contamination-proof stopper, in particular screw cap, for cell culture bottles, is known from WO 88/ 01605. The part of the plastic stopper that covers the bottle opening incorporates a hydrophobic filtering element made of filtering material having a pore size or cut-off that allows sterile gaseous exchange between the bottle content and the ambient atmosphere.

A "reusable vented flask cap cover" is known from US 5,578,491. This document discloses a flask having a vented cap and a plug for sealing the cap when required.

Document US 6,085,922 discloses a "Container and closure assembly with tactile indication of closure" This design is an attempt at solving the problem of tedious manipulation in screwing/unscrewing threaded closures. The closure is rotatable about an axis, whereby the closure is moveable repeatedly back and forth between the open and the closed position.

All these known flasks and adapted closures use some form of threaded cap and corresponding threaded neck. These necks all have, at least at their open end, a circular section. The manual operation of closing, putting in venting position and opening require a rotation of the cap with respect to the flask, which is not the most ergonomic gesture, when performed repeatedly. In addition, although many of the above-cited documents attempt to improve the access to the interior of the flask, by using some special feature, none provide a fully satisfactory solution. Moreover, for opening and closing these threaded caps, some force must be exerted by the cap on the flask. Therefore, the cap cannot be opened or closed with a single hand, on a flask that is not maintained by another hand other means.

Document US 6,114,165 discloses a flask composed of a culture chamber, a wide oblong opening, and a closure unit. The closure unit is constructed with a snap-on-closure, a base and springs, which interconnect the closure and the main body of flask through the base. A first problem with the flasks described in this document is that the presence of folds in the closure is not appropriate for cell cultures. Indeed, when a tool such as a pipette or a scraper enters into the flask, it may happen that cells fall within the folds, which lead to contamination problems when opening the flask again. The use of a spring has further drawbacks. One the one hand, the closure mechanism may show fatigue and may break upon repeated use. On the other hand, as with threaded caps, it is necessary to exert a force onto the cap, which is transmitted to the flask when closing or opening it, in such a way that stacks may be endangered. Another problem is that there is a great risk of imperfect closure, if the user does not exert a sufficient pressure on the top of the closure. Perfect tightness of the mechanism cannot be attained with this kind of flask, which is provided with a small latch and indention system on one side, another side being maintained by the spring. Further, there is no means to control whether the flasks are correctly closed or not. Still another problem is that, due to the fact that the closures are hanging to the flasks when they are open, opening and closing stacked flasks is impossible. The movement that a user must do for closing a flask cannot, in practice, be easily done by using only one hand to rotate said cap from its hanging position and placing it on the flask in such a way that said flask is correctly closed. It is first necessary to take the closure in one hand, to rotate it until it is precisely placed in front of the neck, and thereafter to position it to finally push it on the neck for closure. This requires several operations, possibly to be effected with both hands and is time-consuming. Further, this flask may not be closed in either a vented or air-tight position.

There is therefore a need for a tissue culture flask having a neck providing an easy access to the interior surface of the flask, provided with a cap having both a vented and a sealed position, which is easy to manipulate in the laboratory work. Especially, an easy manipulation of the cap for closing, putting in venting position, or closing the cap is needed, where the open/venting/closed position of the cap can be checked visually and tactually.

### SUMMARY OF THE INVENTION

The present invention overcomes the shortcomings of the conventional art and may achieve other advantages not contemplated by conventional devices.

According to a first aspect of the invention, a combination of laboratory flask and a cap adapted for closure of said flask is provided. Said laboratory flask comprises a chamber and a neck connected to said chamber and giving access to the interior of said chamber. Said cap is made of a deformable elastic material and is adapted for being inserted on said neck by a translation movement along said neck. The exterior surface of said neck and the interior surface of said cap are provided with at least two pairs of respective cooperating means arranged for producing a latching effect when the cap is not deformed and an unlatching effect when the cap is deformed, whereby the cap can be inserted on or removed from said flask by exerting a force on the cap but not on the flask.

Preferably, the respective cooperating means block the cap in a venting position where a passage for gases is left open between said cap and said flask.

A deformation of said cap when in venting position causes a release of said cooperating means, an unlatching thereof.

A pair of cooperating means may comprise a projection on the cap and a recess on the neck or a recess on the cap and a projection on the neck.

Preferably, the combination comprises two pairs of second cooperating means being located opposite each other on the neck-and-cap combination.

According to a preferred embodiment, the cap comprises areas to be depressed for unlatching the cooperating means.

The neck may comprise latches whereas and the cap may comprise corresponding grips. The neck and cap may comprise respective guiding means, for instance guiding tracks on the neck and ribs on the cap.

According to a preferred embodiment, the neck is a cylinder. In the present description, we understand the word "cylinder" in the most general meaning, i.e. a cylinder is the surface generated by a straight line intersecting and moving along a closed plane line, the directrix, while remaining parallel to a fixed straight line that is not on or parallel to the plane of the directrix. More specifically, the directrix is not being limited to a circle but can be any closed curve or closed line. The directrix is also called the section of the cylinder. The neck may have a substantially polygonal section, which may be a polygon with rounded corners or may be a rectangle. The neck may also have a substantially circular section.

According to a second aspect, the invention provides a laboratory flask for use in a combination according to the invention, and a cap adapted for use in such a combination. The cap preferably comprises a skirt adapted for insertion into the neck on a flask, thereby causing a gas- and liquid-tight contact. The skirt preferably comprises a ridge.

The invention also provides a method of opening and closing a laboratory flask by using a combination according to the invention.

Other aspects and advantages of embodiments of the invention will be discussed with reference to the figures and to the detailed description of preferred embodiments.

### BRIEF DESCRIPTION OF THE DRAWINGS

Fig. 1 is a perspective view of a combination of a cap and flask (partial view) according to the invention, wherein the cap and flask are separated.

Fig. 2 is a perspective and partially sectional view of a combination of a cap and flask (partial view) according to the invention, wherein the cap is positioned in closed position.

Fig. 3a, 3b, 3c are respectively a side sectional view, side view, and top view of a combination of a flask and cap according to the invention wherein the cap is in closed position.

Fig. 4a, 4b, 4c are a side sectional view of a part of a combination of a flask (partial view) and cap according to the invention wherein the cap is in respectively venting position, semi-venting position and closed position.

Fig. 5a is a sectional side view along line A-A of fig. 5c of a cap of a combination according to the invention.

Fig. 5b is a sectional side view along line B-B of fig. 5c of a cap of a combination according to the invention.

Fig. 5c is a top view of a cap of a combination according to the invention.

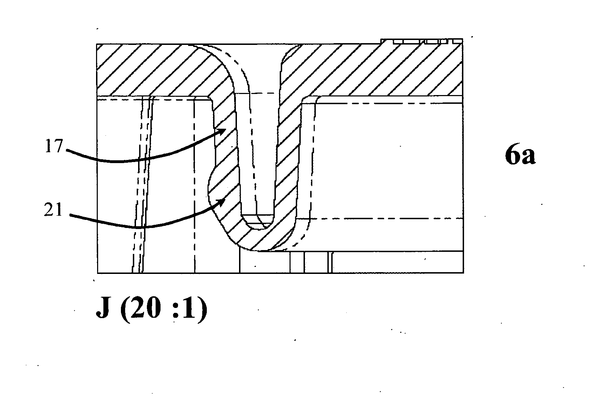

Fig. 6a is a sectional side view along line A-A of Fig. 5a of an enlarged detail of cap of a combination according to the invention.

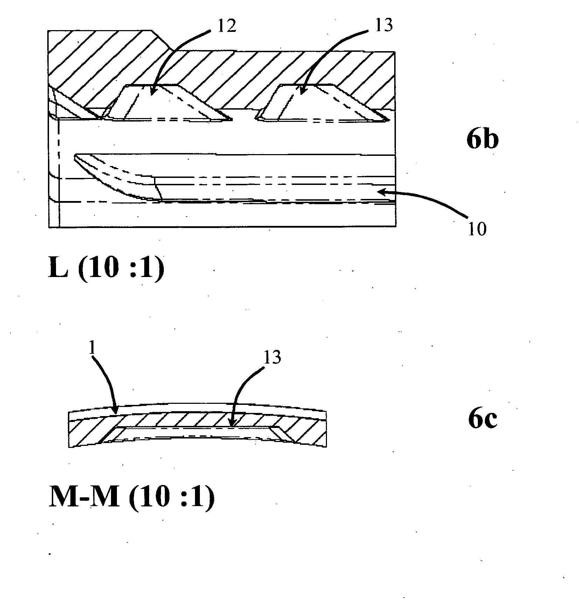

Fig. 6b is a sectional top view of Fig. 5b of an enlarged detail of cap of a combination according to the invention.

Fig. 6c is a sectional top view along line M-M of an enlarged detail of cap of a combination according to the invention.

### DETAILED DESCRIPTION OF THE INVENTION

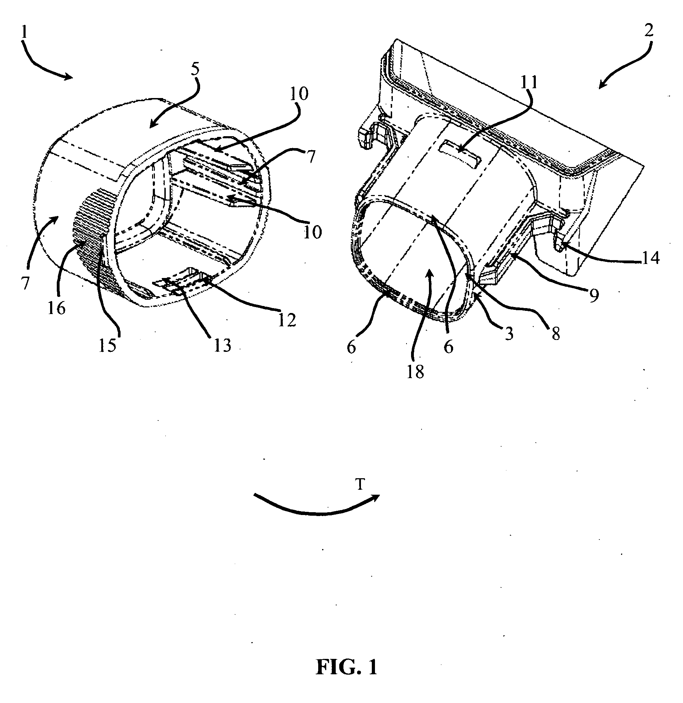

Referring to Fig. 1, a cap (1) and a partial view of a flask (2) are displayed side by side. The cap (1) may be inserted over the neck (3) of the flask (2) by a translational movement along arrow T. Both neck (3) and cap (1) have a substantially rectangular section with rounded corners. When inserting the cap (1) over the neck (3), the longer faces (5) of the cap are oriented towards the longer faces (6) of the neck (3). Guiding tracks (9) are provided on exterior of the shorter faces (8) of neck (3), and corresponding guided ribs (10) are provided on the two opposed interior shorter faces (7) of cap (1) (half of ribs (10) are concealed on the perspective view of Fig. 1). Projections (11) are provided also on the two opposed exterior longer faces (6) of neck (3), and corresponding recesses (12) and (13) are provided on opposed interior faces of cap (1) (half of the recesses (12) and (13) are concealed on the perspective view of Fig. 1).

When closing a flask (2) with a cap (1), an operator starts inserting the cap (1) on the neck (3). In its travel over the neck of the bottle, the cap ribs (10) are guided by guiding tracks (9). When projections (11) face recesses for venting position (12), a tactile resistance is sensed, and a clicking noise is heard, confirming that the cap (1) is in venting position. The operator may then proceed with the insertion up to a position when projections (11) face recesses for closed position (13), when again a tactile resistance is sensed, and a clicking noise is heard, confirming that the cap (1) is in closed position.

For improved security, latches (14) are provided at the end of the guiding tracks (9), and corresponding grips (15) at the exterior of the shorter faces (7) of the cap (1). The cap (1) is made of a deformable material, so that by depressing the opposed shorter faces (7) of the cap (1), the opposed longer faces (5) separate from each other, and recesses (12) or (13) are released from projections (11), and when in closed position, latches (14) are released from grips (15). For ease of use, the area to be depressed (16) is grooved.

When pressing the cap (1) between thumb and index on opposite areas (16), the user pinches it and inserts same without any friction and with no force exerted by the cap (1) on the neck (3). At the end of the movement, the user releases the pressure on the cap (1), which is then blocked on the neck (3) through the cooperation between projections (11) and recess (12). In such a way, one may easily place the cap (1) on the neck (3) with one hand only, using in fact only two fingers. There is no risk to accidentally move the flask during the closing movement. For opening the flask, it is sufficient to pinch the cap (1) again, and simply remove it from the neck, by a translation movement, without exerting any force on the flask.

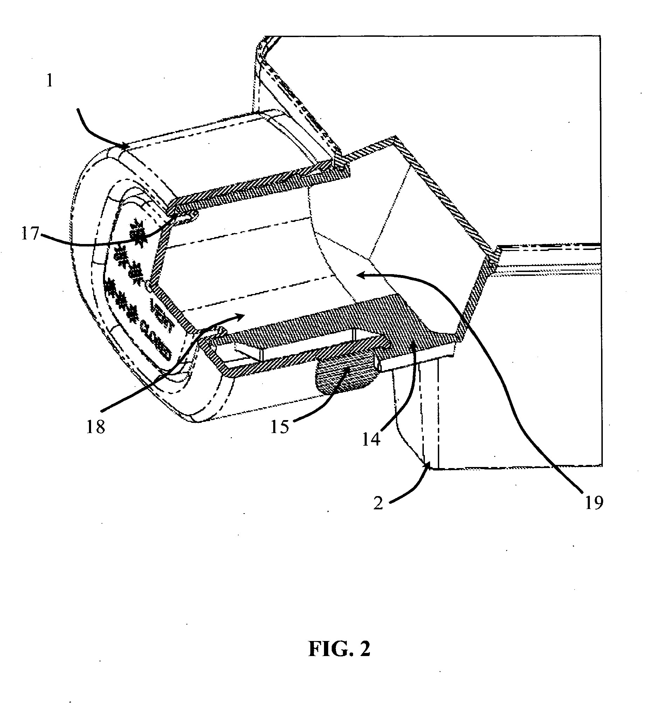

Fig. 2 is a perspective and partially sectional view of a combination of a cap (1) and flask (2) according to the invention, wherein the cap (1) is positioned in closed position. Projections (11) are engaged in recesses for closed position (13), and latches (14) are engaged with grips (15). The upper part of the cap (1) is provided with a skirt (17) which, in closed position, is pressed in contact with the interior surface of neck (3), thereby ensuring tightness of the closure. By using the design of the neck (3) and cap (1) of the invention, with substantially rectangular cross section, a flat part (18) is obtained at the lower part of the neck (3). This flat part (18) intersects continuously with a ramp or inclined plane (19) connected to the bottom wall (20) of the flask. The use of cell scrapers across the bottom of the flask (20), and up the neck (3) of the flask (2) is thereby facilitated. When the user wants to open the flask (2), he pushes with thumb and index on the areas (16), thereby unlatching each pair of projection (11) and recess (13). The cap (1) may be then removed from the neck (3) through a translational movement. No force is exerted on the neck and hence it is not necessary to maintain the flask (2) with the hand or other means during opening, nor during closure.

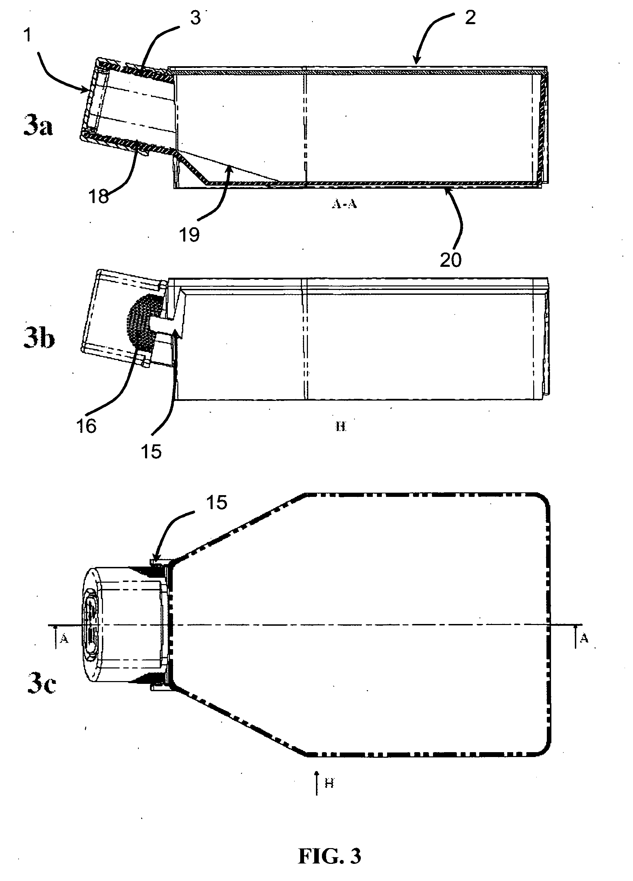

Fig. 3a is a side sectional view of a flask (2) and cap (1) according an embodiment of the invention wherein the cap (1) is in closed position. As shown in the top view, Fig 3c, the flask may have a generally hexagonal shape. A set of flasks of various contents are conventionally made and used in laboratories, ranging from 50 ml to 1000 ml. Fig. 3a shows that flat part (18) of lower part of neck (3) merges continuously with ramp (19), and then with bottom wall (20) of flask. It can be seen that a scraper can easily be moved across this path.

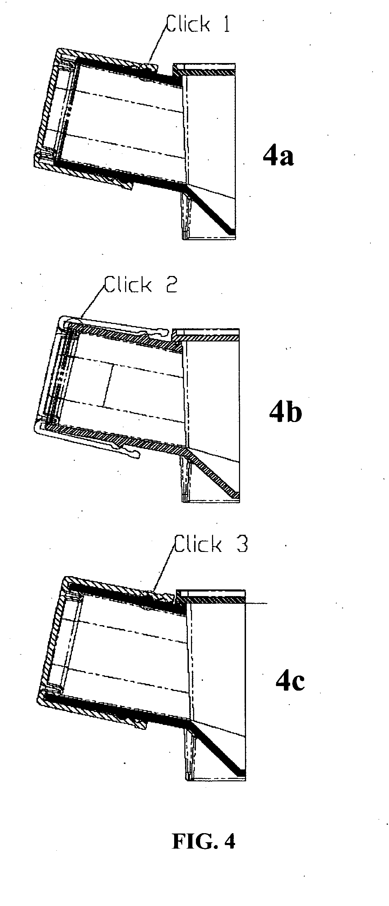

Fig. 4a is a side sectional view of a part of a combination of a flask and cap according to the invention wherein the cap is in venting position. The skirt (17) is not in contact with the interior surface of neck (3). Therefore, a free passage for air or gases is left between the cap (1) and the neck (3). The cap is held firmly in place by the fitting of the two projections (11) in the corresponding two recesses for venting position (12). When inserting cap (1) over neck (3), a first click is heard when reaching the venting position depicted in fig. 4a. This position is attained without exerting any force on the neck (2). When further pushing cap (1) over neck (3), a second click is heard and felt when ridge (21) contacts inner wall of neck (3). This semi-venting position is shown on Fig. 4b, and provides a intermediate venting. When still further pushing cap (1) over neck (3), a third click is heard and felt when projection (11) contacts recess for closed position (13). This closed position is shown on Fig. 4c. As shown on Fig. 2, an indication on these three clicks is marked on the top of the cap.

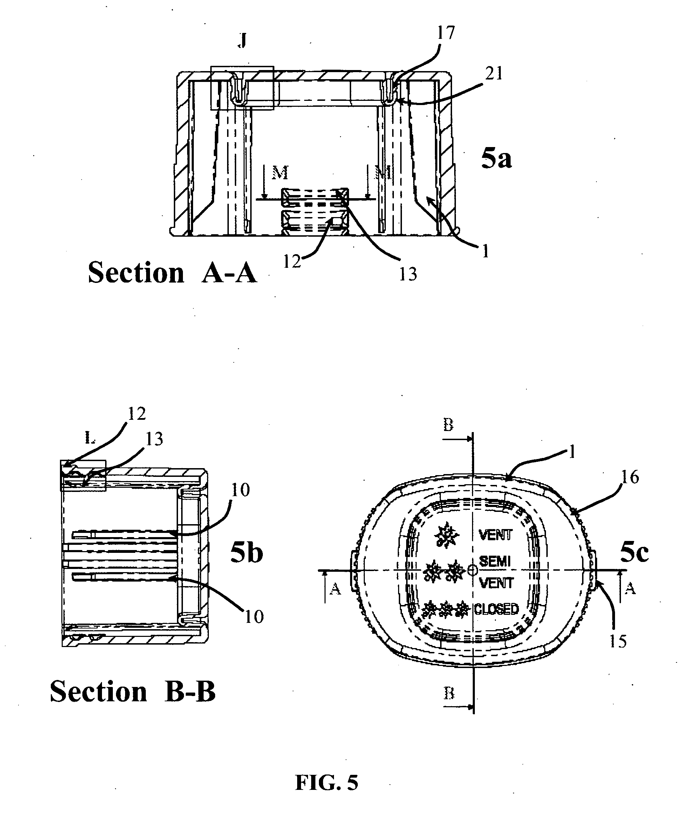

Fig. 5a is a sectional side view of a cap of a combination according to the invention.

Fig. 5b is a sectional side view of an enlarged detail of cap of a combination according to the invention. The face of skirt (17) facing the interior of neck (3) is made slightly conical (e.g. 6 degrees) and provided with a ridge (21). Thereby, the insertion of the skirt (17) into the neck (3) is at first easy, but encounters an increased resistance as progress is made, and as the pressure of ridge (21) against the interior of neck (3) increases. Conversely, when the cap (1) is released from its closed position by depressing grooved areas (16) on the shorter sides (7) of the cap, the cap (1) withdraws easily.

Fig. 5c is a sectional top view along line L-L of Fig. 5a of an enlarged detail of cap of a combination according to the invention and displays the recess for closed position (13) made at the inside part of the longer sides (5) of the cap (1).

Fig. 5d is a sectional top view of Fig. 5b of an enlarged detail of cap of a combination according to the invention and displays the recess for venting position (12) and for closed position (13) made at the inside part of the longer sides (5) of the cap (1). The section of these recesses is made conical with an angle of 30 degrees for providing a latching mechanism with the projections (11).

The flask is preferably made of a clear transparent rigid plastic material such as polystyrene. The cap is preferably made of a more elastic material such as polyethylene and is also preferably colored. Although the neck and cap have been described above as being cylindrical, these may be made slightly conical, e.g. one or two degrees, in order to make the removal out of a mold easier.

Projections (11) and corresponding recesses (12) an (13) may be permuted from neck (3) to cap (1) and conversely. Similarly, guiding tracks (9) may be provided inside the cap (1) and guided ribs (10) on the outside of neck (3). Although the cap and neck polygonal section illustrated in the preferred embodiment is a rectangle, other polygons may be contemplated, such as triangle, square, pentagon, and hexagon.

## Claims

1. A combination of laboratory flask (2) and a cap (1) adapted for closure of said flask (2), wherein said laboratory flask (2) comprises a chamber and a neck (3) connected to said chamber and giving access to the interior of said chamber, **characterized in that** said cap (1) is made of a deformable elastic material and is adapted for being inserted on said neck (3) by a translation movement along said neck (3), the exterior surface of said neck (3) and the interior surface of said cap (1) being provided with at least two pairs of respective cooperating means (11-12, 11-12) arranged for producing a latching effect when the cap (1) is not deformed and an unlatching effect when the cap (1) is deformed, whereby the cap (1) can be inserted on or removed from said flask (2) by exerting a force on the cap (1) but not on the flask (2).

2. A combination according to any of the preceding claims **characterized in that** the respective cooperating means (11, 12) block the cap (1) in a venting position where a passage for gases is left open between said cap (1) and said flask (2).

3. A combination according to any of the preceding claims **characterized in that** a deformation of said cap (1) when in venting position causes a release of said cooperating means (11-12).

4. A combination according to any of the preceding claims, wherein a pair of cooperating means (11-12) comprises a projection (11) on the neck (3) and a recess (12) on the cap (1).

5. A combination according to any of the preceding claims, wherein a pair of cooperating means comprises a recess on the neck (3).and a projection on cap (1).

6. A combination according to any of the preceding claims, **characterized in that** the combination comprises two pairs of cooperating means (11-12, 11-12) being located opposite each other on the neck.

7. A combination according to any of the preceding claims **characterized in that** said flask (2) and said cap (1) respectively comprise at least two pairs of second respective cooperating means (11-13, 11-13) for blocking the cap (1) in a closed position where the closure between said cap (1) and said flask (2) is gas-tight.

8. A combination according to claim 7, **characterized in that** the combination comprises two pairs of second cooperating means (11-13, 11-13) being located opposite each other on the neck-and-cap assembly.

9. A combination according to any of the preceding claims **characterized in that** said neck is a cylinder having a substantially polygonal section.

10. A combination according to any of claims 1 to 9 **characterized in that** the neck (3) has a substantially circular section.

11. A laboratory flask (2) adapted for use in a combination according to any of claims 1 to 10.

12. A cap (1) adapted for use in a combination according to any of claims 1 to 10.

13. A cap (1) according to claim 12 **characterized in that** comprises a skirt (17) adapted for insertion into the neck of a flask (3), thereby causing a gas- and liquid-tight contact.

14. A cap (1) according to claim 13 **characterized in that** said skirt (17) comprises a ridge (21).

15. A method of opening and closing a laboratory flask (2) **characterized in that** it comprises the step of using a combination according to claims 1 to 10.

## Patentansprüche

1. Kombination aus Laborkolben (2) und Deckel (1), geeignet für den Verschluss des Kolbens (2), wobei der Laborkolben (2) eine Kammer und einen Hals (3) umfasst, der mit der Kammer verbunden ist und Zugang zum Inneren der Kammer gewährt, **dadurch gekennzeichnet, dass** der Deckel (1) aus einem verformbaren elastischen Material hergestellt ist und dazu geeignet ist, durch eine translatorische Bewegung in den Hals (3) eingefügt zu werden, wobei die Außenfläche des Halses (3) und die Innenfläche des Deckels (1) mit mindestens zwei Paaren von entsprechenden zusammenarbeitenden Mitteln (11-12, 11-12) versehen sind, die angeordnet sind, um einen Einklinkeffekt zu erzeugen, wenn der Deckel (1) nicht verformt ist, und einen Ausklinkeffekt, wenn der Deckel (1) verformt ist, wobei der Deckel (1) auf dem Kolben (2) eingeführt oder davon entfernt werden kann, indem eine Kraft auf den Deckel (1), jedoch nicht auf den Kolben (2) ausgeübt wird.

2. Kombination nach einem der vorhergehenden Ansprüche, **dadurch gekennzeichnet, dass** die entsprechenden zusammenarbeitenden Mittel (11, 12) den Deckel (1) in einer Lüftungsposition blockieren, wobei ein Durchgang für Gase zwischen dem Deckel (1) und dem Kolben (2) offen gelassen wird.

3. Kombination nach einem der vorhergehenden Ansprüche, **dadurch gekennzeichnet, dass** eine Verformung des Deckels (1) in der Lüftungsposition eine Freigabe der zusammenarbeitenden Mittel (11-12) hervorruft.

4. Kombination nach einem der vorhergehenden Ansprüche, wobei ein Paar von zusammenarbeitenden Mitteln (11-12) einen Vorsprung (11) auf dem Hals (3) und eine Aussparung (12) auf dem Deckel (1) umfasst.

5. Kombination nach einem der vorhergehenden Ansprüche, wobei ein Paar von zusammenarbeitenden Mitteln eine Aussparung auf dem Hals (3) und einen Vorsprung auf dem Deckel (1) umfasst.

6. Kombination nach einem der vorhergehenden Ansprüche, **dadurch gekennzeichnet, dass** die Kombination zwei Paare von zusammenarbeitenden Mitteln (11-12, 11-12) umfasst, die sich einander gegenüberliegend auf dem Hals befinden.

7. Kombination nach einem der vorhergehenden Ansprüche, **dadurch gekennzeichnet, dass** der Kolben (2) bzw. der Deckel (1) mindestens zwei Paare von zweiten entsprechenden zusammenarbeitenden Mitteln (11-13, 11-13) umfassen, um den Deckel (1) in einer geschlossenen Position zu blockieren, wobei der Verschluss zwischen dem Deckel (1) und dem Kolben (2) gasdicht ist.

8. Kombination nach Anspruch 7, **dadurch gekennzeichnet, dass** die Kombination zwei Paare von zweiten zusammenarbeitenden Mitteln (11-13, 11-13) umfasst, die sich einander gegenüberliegend auf der Hals-Deckel-Einheit befinden.

9. Kombination nach einem der vorhergehenden Ansprüche, **dadurch gekennzeichnet, dass** der Hals ein Zylinder mit einem im Wesentlichen polygonalen Querschnitt ist.

10. Kombination nach einem der Ansprüche 1 bis 9, **dadurch gekennzeichnet, dass** der Hals (3) einen im Wesentlichen kreisförmigen Querschnitt aufweist.

11. Laborkolben (2), geeignet für die Verwendung in einer Kombination nach einem der Ansprüche 1 bis 10.

12. Deckel (1), geeignet für die Verwendung in einer Kombination nach einem der Ansprüche 1 bis 10.

13. Deckel (1) nach Anspruch 12, **dadurch gekennzeichnet, dass** er einen Randabschluss (17) aufweist, der dazu geeignet ist, in den Hals eines Kolbens (3) eingeführt zu werden, um dabei einen gas- und flüssigkeitsdichten Kontakt hervorzurufen.

14. Deckel (1) nach Anspruch 13, **dadurch gekennzeichnet, dass** der Randabschluss (17) einen Wulst (21) umfasst.

15. Verfahren zum Öffnen und Schließen eines Laborkolbens (2), **dadurch gekennzeichnet, dass** es den Schritt des Verwendens einer Kombination nach Anspruch 1 bis 10 umfasst.

## Revendications

1. Une combinaison d'un flacon de laboratoire (2) et d'un capuchon (1) adapté pour la fermeture dudit flacon (2), dans laquelle ledit flacon de laboratoire (2) comprend une chambre et une encolure (3) connectée à ladite chambre et donnant accès à l'intérieur de ladite chambre, **caractérisée en ce que** ledit capuchon (1) est fabriqué en un matériau élastique déformable et est adapté pour être inséré sur ladite encolure (3) par un mouvement de translation le long de cette encolure (3), la surface extérieure de ladite encolure (3) et la surface intérieure dudit capuchon (1) étant munie d'au moins deux paires de moyens de coopération (11-12, 11-12) disposés pour produire un effet de verrouillage lorsque le capuchon (1) n'est pas déformé et un effet de déverrouillage lorsque le capuchon (1) est déformé, par lequel le capuchon (1) peut être inséré sur ou retiré dudit flacon (2) en exerçant une force sur le capuchon (1) mais pas sur le flacon (2).

2. Une combinaison selon l'une quelconque des revendications précédentes, **caractérisée en ce que** les moyens de coopération (11, 12) bloquent le capuchon (1) dans une position de ventilation où un passage pour les gaz est laissé ouvert entre ledit capuchon (1) et ledit flacon (2).

3. Une combinaison selon l'une quelconque des revendications précédentes, **caractérisée en ce qu'**une déformation dudit capuchon (1) en position d'aération provoque un relâchement desdits moyens de coopération (11-12).

4. Une combinaison selon l'une quelconque des revendications précédentes, dans laquelle une paire de moyens de coopération (11-12) comprend une projection (11) sur l'encolure (3) et un renfoncement (12) sur le capuchon (1).

5. Une combinaison selon l'une quelconque des revendications précédentes, dans laquelle une paire de moyens de coopération comprend un renfoncement sur l'encolure (3) et une projection sur le capuchon (1).

6. Une combinaison selon l'une quelconque des revendications précédentes, **caractérisée en ce que** la combinaison comprend deux paires de moyens de coopération (11-12, 11-12) qui sont situés à l'opposé l'un de l'autre sur l'encolure.

7. Une combinaison selon l'une quelconque des revendications précédentes, **caractérisée en ce que** ledit flacon (2) et ledit capuchon (1) comprennent respectivement au moins deux paires de second moyens de coopération (11-13, 11-13) pour bloquer le capuchon (1) dans une position fermée, où la fermeture entre ledit capuchon (1) et ledit flacon (2) est étanche aux gaz.

8. Une combinaison selon la revendication 7, **caractérisée en ce que** la combinaison comprend deux paires de seconds moyens de coopération (11-13, 11-13) qui sont localisés à l'opposé l'un de l'autre sur l'ensemble encolure et bouchon.

9. Une combinaison selon l'une quelconque des revendications précédentes **caractérisée en ce que** ladite encolure est un cylindre ayant une section substantiellement polygonale.

10. Une combinaison selon l'une quelconque des revendications 1 à 9, **caractérisée en ce que** l'encolure (3) a une section substantiellement circulaire.

11. Un flacon de laboratoire (2) adapté pour une utilisation dans une combinaison selon l'une des revendications 1 à 10.

12. Un capuchon (1) adapté pour une utilisation dans une combinaison selon l'une des revendications 1 à 10.

13. Un capuchon (1) selon la revendication 12, **caractérisé en ce qu'**il comprend une jupe (17) adaptée pour l'insertion dans l'encolure du flacon (3), provoquant ainsi un contact étanche aux liquides et aux gaz.

14. Un capuchon (1) selon la revendication 13 **caractérisé en ce que** ladite jupe (17) comprend une arête (21).

15. Une méthode pour ouvrir et fermer un flacon de laboratoire (2) **caractérisée en ce qu'**elle comprend l'étape d'utiliser une combinaison selon les revendications 1 à 10.
